(19) Europäisches Patentamt
European Patent Office
Office européen des brevets

(11) **EP 4 336 517 A1**

(12) **EUROPEAN PATENT APPLICATION**

(43) Date of publication:
**13.03.2024 Bulletin 2024/11**

(21) Application number: **22194751.8**

(22) Date of filing: **09.09.2022**

(51) International Patent Classification (IPC):
**G16H 50/30** (2018.01)    **G16H 50/50** (2018.01)

(52) Cooperative Patent Classification (CPC):
**G16H 50/50; G16H 50/30**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Université de Genève - UNIGE**
**1211 Genève (CH)**
• **Ecole Polytechnique Fédérale de Lausanne (EPFL)**
**1015 Lausanne (CH)**
• **Hopitaux Universitaires de Genève**
**1205 Genève (CH)**

(72) Inventors:
• **Rivier, Cyprien**
**New Haven, 06510 (US)**
• **Pirondini, Elvira**
**Pittsburg, 15206 (US)**
• **Guggisberg, Adrian**
**1211 Genève (CH)**
• **Van de Ville, Dimitri**
**1166 Perroy (CH)**

(74) Representative: **KATZAROV S.A.**
**Geneva Business Center**
**12 Avenue des Morgines**
**1213 Petit-Lancy (CH)**

(54) **POST STROKE RECOVERY PREDICTION**

(57) The present relates to a method for generating a graph representing a virtually lesioned connectome of a patient after a stroke comprising:
- providing structural data of the brain of the patient after the stroke;
- segmenting said structural data to delineate lesioned voxels and non-lesioned voxels to generate a lesion map;
- providing at least one connectome of a healthy reference brain, said connectome representing white matter fibers of the said healthy reference brain;
- overlapping the lesion map on the connectome and removing each white matter fiber of the connectome passing through one lesioned voxel so as to generate a virtually lesioned connectome;
- constructing a graph for each virtually lesioned connectome by parcellating the virtually lesioned connectome into N region of interest, each region of interest corresponding to a vertex of the graph, the edges connecting two vertices representing the fibers connecting two regions of interest;

Figure 1

**Description**

**Technical Field**

The present invention relates to a method for generating a graph representing a virtually lesioned connectome of a patient after a stroke. The invention also relates to a method for predicting the recovery of a patient after a determined period following a stroke, preferably based on the graph generated by the method according to the present invention.
The invention further relates to a computer program comprising instructions, which, when the program is executed by a computer, cause the computer to carry out the methods according to the present invention.

**Background of the art**

In the United States alone, almost 800,000 people are affected every year by a cerebral stroke with consequences that include severe motor, cognitive, or emotional handicap. Unfortunately, less than 15% of the patients achieve full recovery, making the number of persons currently living in the US with motor impairments as a consequence of stroke on the order of millions. An additional challenge is the heterogeneity in outcome and individual recovery potential that strongly influence our ability to identify the optimal neurorehabilitative program to maximize individual treatment outcome. Indeed, even experienced clinicians find it difficult to accurately predict or evaluate patients' level of recovery. Accurate prediction in the early stage after the lesion has the potential to yield a patient-specific recovery trajectory, stratify patients into recovery-focused clinical trials, and guide rehabilitation strategies including discharge destination.
It has already been shown that the severity of motor impairment at admission provides useful information on the recovery trajectory. Indeed, the comparison of the Fugl-Meyer score obtained right after the stroke and 3 to 6 months later led to the postulation of the proportional recovery model, which states that the majority of patients recover on average 70% of their initial impairments.
However, models which aim to predict a percentage of the initial impairment using the initial impairment as an independent variable lead to an overestimation of the fit between initial impairment and clinical recovery due to mathematical coupling. Moreover, the 70% rule does not apply to all patients. Indeed, even though the recovery trajectory of about 2/3 of the patients fits this so-called proportional rule, the remaining ones, the so-called non-fitters, show very poor to no improvement. More recent work avoiding mathematical coupling confirmed that patients present two distinct recovery patterns, and that the clinical recovery shows proportionality to the initial impairment, although with much less explained variance. Prediction of motor recovery remains, thus, an important and currently unmet need.
The integrity of the cortico-spinal tract, as assessed with brain imaging or with motor evoked potentials, has also been proposed as a good predictor of motor recovery after stroke. Indeed, large cortico-spinal tract (CST) lesions, as depicted by a high degree of CST asymmetry, are linked to poor motor improvement. Yet, a combination of clinical assessments and measures of CST integrity allow a reliable prediction of motor recovery in only 75% of patients.
The lack of generalization of these prediction models might be explained by the use of measures derived purely from focal brain regions. Indeed, increasing evidence settled a new clinical concept in stroke: connectional diaschisis, which illustrates the idea that even a focal lesion might induce a loss of functionality in a territory that is distant to the lesion. This observation is not surprising if we consider the brain as one complex network where the different regions are nodes linked together by a global architecture that makes them dependent on one another.
For this reason, recent studies attempted to include in the prediction models network measures extracted from functional and diffusion-weighted magnetic resonance imaging (MRI) with encouraging results. However, measuring functional and diffusion-weighted MRI signals require long and expensive acquisitions that are not easily implementable in clinical settings particularly in the acute phase post-lesion.
Therefore, there is a need to provide a solution to facilitate the prediction or evaluation of the patient's recovery after a determined period of time following the stroke and overcome the issues of the existing approaches.

**Summary of the invention**

The above problems are solved or at least minimized by the present invention.
This object has been achieved by providing a method, preferably a computer implemented method, for generating a graph representing a virtually lesioned connectome of a patient after a stroke, the method comprising

- providing structural data of the brain of the patient after the stroke;

- segmenting said structural data to delineate lesioned voxels and non-lesioned voxels so as to generate a lesion map;

- providing at least one connectome of a healthy reference brain, said connectome representing white matter fibers of the said healthy brain;

- overlapping the lesion map on the connectome and removing each white matter fiber of the connectome passing through one lesioned voxel so as to generate a virtually lesioned connectome;

- constructing a graph for each virtually lesioned connectome by parcellating the virtually lesioned connectome cortex into N region of interest, each region of interest corresponding to a vertex of the graph, the edges connecting two vertices representing the fibers connecting two regions of interest;

Another object of the present invention is to provide a method, preferably a computer implemented method, for predicting the recovery of a patient after a determined period following a stroke, the method comprises

- providing a set of features measured from the patient after the stroke;

- providing a computational model configured for predicting the recovery of a patient after a determined period following a stroke;

- using said set of features as input for the computational model to calculate a recovery score as output to predict the recovery of the patient;

the method being characterized in that the set of features comprises brain connectivity measures, said brain connectivity measures being computed from at least one graph generated by a method for generating a graph according to the present invention.

A further object of the present invention is to provide device comprising means for carrying out the method of the present invention, preferably the device is a computer device.

A further object of the present invention is to provide a computer program comprising instructions, which, when the program is executed by a computer, cause the computer to carry out the method of the present invention.

**Description of the invention**

In a first aspect, the invention concerns a computer-implemented method for generating a graph representing a virtually lesioned connectome of a patient after a stroke, the method comprising

- providing structural data of the brain of the patient after the stroke;

- segmenting said structural data to delineate lesioned voxels and non-lesioned voxels so as to generate a lesion map;

- providing at least one connectome of a healthy reference brain, said connectome representing white matter fibers of the said healthy reference brain;

- overlapping the lesion map on the connectome and removing each white matter fiber of the connectome passing through one lesioned voxel so as to generate a virtually lesioned connectome;

- constructing a graph for each virtually lesioned connectome by parcellating the virtually lesioned connectome into N region of interest, each region of interest corresponding to a vertex of the graph, the edges connecting two vertices representing the fibers connecting two regions of interest;

The logic behind the present invention is that it uses a graph to summarize in a few or limited number of measures values from several brain regions. The use of graph further allows to capture interactions between brain areas to enhance or facilitate the understanding of the cortical reorganization after stroke.

Advantageously, the graph of the present invention is constructed from connectome of white matter fibers from a healthy reference brain whereas generally graph are constructed solely from functional MRI (fMRI). The advantage to use a graph constructed from white matterfibers compared to fMRI is that 1) the acquisitions are much faster for diffusion images used to reconstruct white matter tracts than acquisition of fMRI data; and 2) the computational algorithm to pre-process the data and extract the connectome is lighter for diffusion images than for fMRI images. In other words, the combination of white fibers matter images and structural data facilitate the construction of the graph compared to existing graph construction methods.

Preferably, the graph of the present invention is constructed from connectome of white matter fibers from a healthy reference brain and from functional MRI (fMRI) from a healthy reference brain.

Advantageously, the measures of brain connectivity estimated by introducing virtual lesions in healthy structural connectomes significantly increase the accuracy of motor recovery prediction in stroke patients compared to focal and clinical measures used in existing methods until now.

In the present invention, the prediction of the recovery of the patient after a stroke is calculated by taking into account brain connectivity measures computed from a virtual lesioned connectome. This indirect estimation of the stroke impact on the whole brain connectome is more readily available than direct measures of structural connectivity obtained with magnetic resonance imaging (MRI).

In a preferred embodiment, a plurality of connectomes from healthy reference brains is provided, each connectome being used to generate one virtually lesioned connectome, and each virtually lesioned connectome is used to construct one graph. If each graph is used to construct a virtually lesioned connectome, this allows to prepare multiple virtually lesioned connectomes. Using multiple virtually lesioned connectomes allows to reduce the variability on the brain connectivity measures. This also minimizes or avoids spurious values that could be due to a particular reference brain, the spurious values would lead to a less precise prediction.

Preferably, the connectome from the healthy reference brain can be a connectome from the same patient who had the stoke, but before the stroke.

Preferably, when several graphs are constructed, the method comprises:

- Averaging the shared connectivity measures among the graphs to obtain an average value for each shared connectivity measure;

This is advantageous because using several healthy reference templates reduces the effects of the interindividual variability in white matter microstructure on the brain connectivity measures. A higher count of reference connectomes is advantageous to increase the stability of the connectivity measures. Using multiple virtually lesioned connectome allow to reduce the variability on the brain connectivity measures thereby limiting or avoiding spurious values that could be due to a particular healthy reference brain.

Preferably, the method comprises

- computing brain connectivity measures from the generated graph for instance binary graph measures and/or weighted graph measures, preferably binary graph measures chosen form the list comprising density, median degree, mean clustering coefficient, median clustering coefficient, mean flow coefficient, transitivity, modularity, mean eigenvector centrality, median eigenvector centrality, mean betweenness centrality, median betweenness centrality, preferably weighted graph measures preferably chosen form the list comprising algebraic connectivity, mean eigenvector centrality, median eigenvector centrality, modularity, global efficiency, participation coefficient, quasi-idempotence,

In a preferred embodiment, the brain connectivity measures comprise modularity weighted, participant coefficient, transitivity, median eigenvector centrality and mean eigenvector centrality weighted. These measures were selected from the computable binary graph measures and/or weighted graph measures. This selection of connectivity measures was carried out using rigorous feature selection in an existing dataset. This set of features was stable over the cross-validation folds of this existing dataset. Advantageously, this selection of connectivity measures proves to generalize well to other datasets.

Preferably the structural data comprise data chosen from the list comprising MRI data such as T2-weighted, T1-weighted, diffusion-weighted, computerized tomography (CT).

Preferably, the segmenting step is performed automatically for instance by using an automated segmenting tool or manually by an operator.

Preferably, the lesioned map corresponds to three dimensions 3D map of zeros for non-lesioned voxels and ones for lesioned voxels.

Another object of the present invention is to provide a method, preferably a computer implemented method, for predicting the recovery of a patient after a determined period following a stroke, the method comprises

- providing a set of features measured from the patient after the stroke;

- providing a computational model configured for predicting the recovery of a patient after a determined period following a stroke;

- using said set of features as input for the computational model to calculate a recovery score as output to predict the recovery of the patient;

the method being characterized in that the set of features comprises brain connectivity measures, said brain connectivity measures being computed from at least one graph corresponding to a virtual lesioned connectome of the patient generated by a method for generating a graph according to the present invention.

In the present invention, the lesioned connectome of the patient after stroke is virtually generated. In other words, it avoids acquiring diffusion images of the patient after stroke and so it makes the acquisitions more clinically possible. Most of the recent method for predicting stroke recovery included measurements obtained by neuroimaging or neurophysiological recordings that better capture interindividual lesion-induced neural abnormalities. However, these neuro-biomarkers are obtained by special imaging acquisitions such as functional MRI or diffusion-weighted imaging that require computationally expensive algorithms of analysis and prediction, thus posing several challenges for practical application in clinical routine.

Here the proposed computationally-efficient prediction solution which does not require special imaging acquisitions such as functional MRI or diffusion-weighted imaging. The present invention leverages high-quality tractography data, for instance from a public dataset in healthy individuals, and combines them with the patient's lesion mask without the need for diffusion-weighted MRI acquisitions. For instance, the prediction method according to the present invention can be realized in about 10 minutes for the predictions starting from the lesion mask when run with a computer with standard processing power (for instance 12 cores amd ryzen 5900).

Preferably, the parcellation is performed by using a brain parcellation atlas, such as the Glasser's multimodal cortical atlas, the Automated Anatomical Labeling atlas, the JuBrain / Juelich histological atlas.

Preferably, the number of regions of interest N is comprised between about 10 to about 250, more preferably between about 20 to about 200, more preferably between about 30 to about 190, more preferably between about 40 to about 180.

Preferably, the method further comprises

Selecting a group of features among the set of features, the selection being carried out using selection tool such as variance inflating factors (VIF), LASSO, or hierarchical clustering, preferably variance inflating factors.

In a preferred embodiment, the recovery score is chosen among the list comprising Fugl-Meyer Assessment (FMA) scale, Stroke Impact Assessment Scale, the Chedoke-McMaster Stroke Assessment, or the Motor Assessment Scale, preferably Fugl-Meyer Assessment scale.

In a preferred embodiment, computational model is chosen among the list comprising ridge regression, Multilinear regression, Lasso, Elastic Net, Bayesian Ridge, preferably ridge regression.

Preferably, the input for the computational model correspond to a set of standardized features to obtain a mean of 0 and a standard deviation of 1.

In a preferred embodiment, the setoff features further comprises features chosen from the list comprising patient's age, initial motor impairment, lesion volume, cortico spinal tract asymmetry.

**Brief description of the drawings**

Further particular advantages and features of the invention will become more apparent from the following non-limitative description of at least one embodiment of the invention which will refer to the accompanying figures, wherein

- Figure 1 illustrates a first embodiment of the present invention;

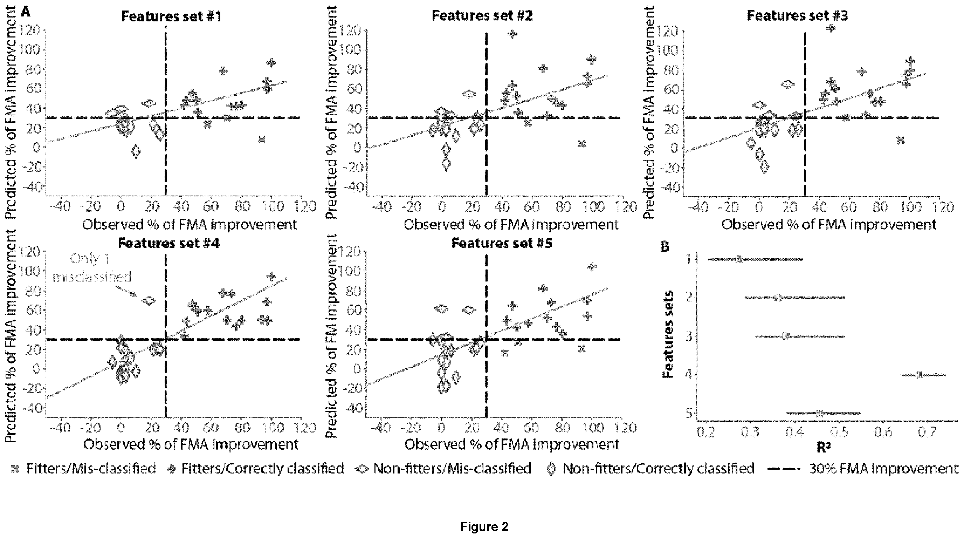

- Figure 2 illustrates the prediction accuracy of the method for predicting the recovery according to the first embodiment.

**Detailed description of the invention**

The present detailed description is intended to illustrate the invention in a non-limitative manner since any feature of an embodiment may be combined with any other feature of a different embodiment in an advantageous manner.

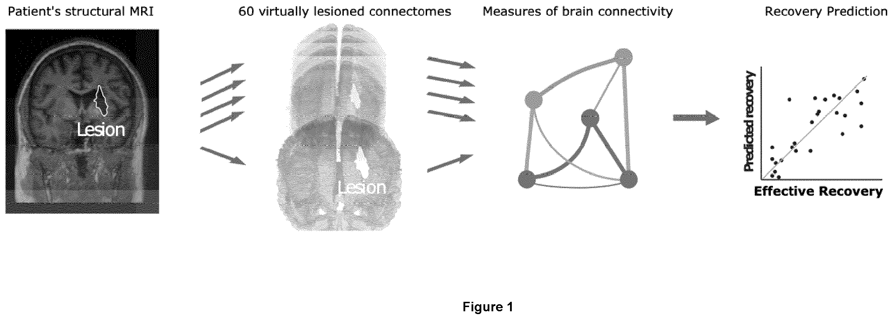

The overall principle of the present invention is represented in figure 1. The main steps are the followings;

- a lesion mask is drawn for each patient from the structural MRI;

- for each patient, the lesion mask is intersected with each of the 60 healthy streamline tractography connectomes deleting all white-matter tracts passing through the lesioned area leading to 60 virtually lesioned connectomes;

- from each of these virtually lesioned connectomes, we estimate brain connectivity measures and the latter are averaged over connectomes.

- these measures are used as input for a ridge regression model (i.e. computation model) to predict motor improvement (i.e. recovery score).

In the present example, 37 subjects were included in this study (dataset #1). Additionally, to obtain a normative structural connectome, we used diffusion-weighted MRI acquisitions from 60 healthy volunteers (dataset #2)

Dataset #1: 37 patients were recruited from the inpatient rehabilitation unit of the University Hospital of Geneva. Inclusion criteria were: (i) clinical diagnosis of stroke involving the territory of the middle cerebral artery as demonstrated by structural MRI; (ii) at least mild motor impairment (upper Fugl-Meyer assessment of at most 55 points) at the beginning of rehabilitation. 63 patients were included in the initial study. The selection of 37 patients (mean age was 65.6, range 28-85) for the present study has then been made on the basis of data completeness, which consists of the presence for each patient of: (i) a diffusion-weighted and T2-weighted imaging obtained 2 to 4 weeks after the stroke for lesion segmentation; (ii) upper FMA at 2 weeks and 3 months after the stroke. Patients for which one of these elements were lacking have been excluded from the present group. All patients received standard therapy at the stroke unit during the acute phase and an individually tailored multidisciplinary rehabilitation program in the subacute and chronic phases. All patients received 2 times 30 minutes of physical therapy per day on 5 days per week and 5 times 30 minutes of occupational therapy per week on an inpatient basis for 8 to 16 weeks, followed by outpatient treatment of 1 to 4 hours of physical and occupational therapy per week.

Clinical assessments: Trained physical or occupational therapists performed standardized clinical assessments of upper extremity motor function at 2-4 weeks and 3 months after stroke using the upper extremity items of the Fugl-Meyer (FMA) with a maximal score of 66 points. We computed the FMA improvement score as: $100 \cdot \frac{FMA_{3months} - FMA_{2weeks}}{66 - FMA_{2weeks}}$, where $FMA_{3months}$ and $FMA_{2weeks}$ are the FMA score at 3 months and 2 weeks post-lesion, respectively.

Magnetic Resonance Imaging acquisition: For both datasets, standard anatomical images and diffusion-weighted images were acquired.

Dataset #1: High-resolution structural T2- weighted (echo time/ repetition time = 376 ms/5.0 s; voxel size = $0.45 \times 0.45 \times 0.90$ mm3) volumes were obtained on a 3.0-T Siemens Trio TIM 3.0T scanner using a 64-channel coil. Additionally, whole brain, single-shot echo-planar (EPI) diffusion-weighted volumes (30 noncollinear directions; b = 1000 s/mm2; 64 slices; voxel size $1.8 \times 1.8 \times 2.0$ mm3; echo time/repetition time = 82 ms/8.2 s; acquisition time = 4 min 40 s) plus one volume without diffusion weighting (b = 0 s/mm2) were acquired parallel to a line intersecting the anterior and posterior commissure.

Dataset #2 : High-resolution structural MRI were acquired using a 3D MPRAGE T1-weighted (TR = 2400 ms, TE = 2.14 ms, TI = 1000 ms, flip angle = 8°, FOV = 224 × 224, voxel size = 0.7 mm isotropic). The following sequence was used for diffusion-weighted MRI: spin-echo EPI, TR = 5520 ms, TE = 89.5 ms, flip angle = 78°, FOV = 208 × 180, 3 shells of b = 1000, 2000, 3000 s mm-2with 90 directions plus 6 b = 0 acquisitions; total acquisition time was 9 min 50s.

Segmentation of the lesion: A lesion mask is drawn for each patient from the structural MRI. In the present example, the method uses structural MRI (e.g., T2-weighted, T1-weighted, diffusion-weighted) to delineate the voxels that are lesioned by the stroke. The delineation of the lesioned voxels (i.e., segmentation) can be done manually by the user or automatically using a software or an algorithm. The segmentation is performed for each stroke patient. This step results in a 3D map of zeros ("non-lesioned" voxel) and ones ("lesioned voxel) per subject.

Identification of damaged fibers: This lesioned map is then overlapped to the connectomes (i.e., a map of white matter fibers as healthy reference brain) obtained from diffusion-weighted MRI of 60 healthy subjects. Once the map is overlapped we assume that each fiber passing through a lesioned voxel is damaged and it is therefore removed from the connectome. This indirect estimation of the stroke impact on the whole brain connectome is more readily available than direct measures of functional and structural connectivity obtained with MRI and utilized in previous method. For each patient, we thus obtain 60 virtually lesioned connectomes. For this step, it is possible to use any set of connectomes from healthy subjects, and in the resent example it is 60 subjects (i.e., it can be more or less).

Construction of the graph: From these virtually lesioned connectomes, we then estimate a graph. For this we use a Glasser's multimodal cortical atlas converted to volume split into the two hemispheres (first 180 areas on the left and last 180 on the right) to parcellate the cortex into N=360 regions of interest. It is important to highlight that other atlases could be used for this parcellation. Examples of such atlases include: Automated Anatomical Labeling (Tzourio-Mazoyer 2002); AICHA, An atlas of intrinsic connectivity of homotopic areas (Joliot 2015); JuBrain / Juelich histological atlas (Eickhoff 2005). Each region of interest corresponds to a node of the graph and the number of fibers connecting two regions divided by the region volumes (sum of connected regions) are the edges connecting two vertices. For each patient, we construct a graph for each virtually lesioned connectome (i.e., in total 60 connectomes per patient in the present example).

Extraction of the brain connectivity measures: From these graphs, we compute seven brain connectivity measures considering the weights of the edges (i.e., weighted graph) and ten measures on binary graphs (i.e., graphs with edges either 0 or 1, see below for details). Some measures are not represented by a single value for the whole network, but by one value for each of the 360 nodes. When this is the case, we consider both the mean and the median to obtain one global value. For each patient and each connectivity measure, we then average the 60 values obtained from the 60 virtually lesioned connectomes. To compute binary measures, the weighted adjacency matrices are first binarized through a thresholding process. As the threshold is inversely proportional to the density of the binarized graph, this must be chosen with care. If the density is too low, the graph is too sparse and some points will be disconnected from the rest of the network. On the other hand, a too high density will lead to too many edges being present, which makes the interpretation of the graph patterns irrelevant. In our case, a threshold is chosen to obtain a density equal to the weighted density of the weighted matrices.

Binary graph measures considered are preferably at least one of density, median degree, mean clustering coefficient, median clustering coefficient, mean flow coefficient, transitivity, modularity, mean eigenvector centrality, median eigenvector centrality, mean betweenness centrality, median betweenness centrality.

Weighted graph measures considered are preferably at least one of algebraic connectivity, mean eigenvector centrality, median eigenvector centrality, modularity, global efficiency, participation coefficient, quasi-idempotence.

Connectivity brain measures selection: Due to the number of connectivity features included, and to avoid collinearity issues in the training part of the prediction model, a feature selection step using variance inflating factors (VIF) is carried out in the present example to select the most important features.

In the present example, the selected connectivity measures from the VIF method on leave-one-out cross-validation folds are the following: weighted modularity, participation coefficient, median eigenvector centrality, transitivity, weighted mean eigenvector centrality, modularity, algebraic connectivity, mean betweenness centrality, quasi-idempotence, median clustering coefficient.

Prediction model: The selected connectivity measures are then considered together with patient's age, initial motor impairment, lesion volume, and cortico-spinal tract asymmetry as features to predict the recovery to prepare the set of features used for predicting stroke recovery. The selected features were standardized to obtain a mean of 0 and a standard deviation of 1, and then used as input for a ridge regression model to predict FMA improvement **(Fig. 1).**

$$Ridge\ regression : min_\beta \left\{ \frac{1}{N} \|y - X\beta\|_2^2 + \lambda_2 \|\beta\|_2^2 \right\}$$

In the present exemple, ridge regression is used as computational model to calculate a recovery score from the selected features, and the FMA improvement is used as recovery score, but the invention is not limited to these choices.

We then use these brain connectivity measures to predict the amount of recovery at three months after the stroke.

The recovery is defined as $100 \cdot \frac{FMA_{3months} - FMA_{2weeks}}{66 - FMA_{2weeks}}$, where $FMA_{3months}$ and $FMA_{2week}$, are the FMA score at 3 months and 2 weeks post-lesion, respectively. FMA is the upper extremity items of the Fugl-Meyer scale.

However, other clinical scores could be used, such as the Stroke Impact Scale, the Chedoke-McMaster Stroke Assessment, or the Motor Assessment Scale.

In order to test whether brain connectivity measures will improve the prediction of recovery as compared to clinical state-of-the-art measures, we compared the predictive power of five different sets of features (Table 1).

**Table 1:** List of sets of features tested.

| Models\Features | Age + Initial FMA | Volume | CST Asymmetry | Brain connectivity |
|---|---|---|---|---|
| *Set 1* | x | | | |
| Set 2 | x | x | | |
| Set 3 (Benchmark) | x | x | x | |
| Set 4 | x | x | x | x |
| Set 5 | x | x | | x |

The first set of features was composed of immediately available information in the clinical setting: patient's age and initial FMA impairment. In the second set of features, we added the lesion volume. The CST asymmetry value was then added to obtain the third set of features. This set of features is the one that so far in literature demonstrated the highest accuracy in predicting motor recovery. In the fourth dataset we added the brain connectivity measures to the other features. Due to the number of connectivity features included, and to avoid collinearity issues in the training part of the prediction model, a feature selection step using variance inflating factors (VIF) was carried out to select the most important features. Finally, in the fifth set of features we excluded CST asymmetry (i.e., we considered only patient's age, initial FMA impairment, lesion volume, and brain connectivity measures). Indeed, CST asymmetry requires advanced imaging acquisitions (as diffusion-weighted MRI) that are not always available in clinical setting. Also, for the fifth set of features, the most predictive brain connectivity measures were selected using VIF.

To test prediction and in particular to test whether brain connectivity measures would lead to a better prediction than benchmark features so far utilized (i.e., patient's age, initial FMA impairment, lesion volume, and CST asymmetry), we built 5 different linear regressor models using 5 different set of features (Table 2). The results are summarized in table 2 below:

**Table 2:** R2, specificity, sensitivity, and positive and negative predictive value for proportional recovery from the five sets of features. Highlighted in blue the set of features with the best prediction.

| Features | $R^2$ | Specificity | Sensitivity | Positive predictive value | Negative predictive value |
|---|---|---|---|---|---|
| *Set 1* | 0.27 | 0.82 | 0.76 | 0.81 | 0.78 |
| Set 2 | 0.36 | 0.88 | 0.76 | 0.87 | 0.79 |
| Set 3 (Benchmark) | 0.38 | 0.88 | 0.76 | 0.87 | 0.79 |
| Set 4 | 0.68 | 1.00 | 0.94 | 1.00 | 0.94 |
| Set 5 | 0.46 | 0.82 | 0.76 | 0.81 | 0.78 |

In particular, computational model accuracy is assessed using the coefficient of determination:

$$R^2 = 1 - \frac{\Sigma(Y-Y')^2}{\Sigma(Y-\overline{Y'})^2}$$

, where *Y* are the measured FMA improvement, *Y'* are the predicted FMA improvement and $\overline{Y'}$ is the mean of predicted FMA improvement scores.

In order to demonstrate the classification ability, i.e., ability to obtain a categorial classification of the patients (0-1) of the five different sets of features, we divided the patients in fitters and non-fitters following the proportional recovery rule as in Koch et al., 2021. Specifically, fitters/non-fitters are patients that follow/not the proportional recovery rule. It has been demonstrated that this separation into 2 classes is robust and not an artifact of mathematical coupling, as it was present also when using Bayesian modelling. In order to determine these two groups of patients, we performed a hierarchical clustering using Spearman correlation distance on the values of FMA points that should be recovered following the 70% proportional recovery model versus the actual number of FMA points effectively recovered by each patient. We additionally used a simple threshold of 30% FMA recovery score to separate fitter from non-fitters, based on literature, and we confirmed that this led to precisely the same labelling as obtained from the hierarchical clustering method. Once we obtained this classification of the patients, we compared each set of features based on their ability to distinguish between fitter and non-fitter using specificity, sensitivity, positive predictive value (PPV), and negative predictive value (NPV).

As expected from previous work, clinical and demographic features (i.e., patient's age and initial FMA impairment) led to good prediction (R2: 0.27, set 1, Table 2 and Fig. 3). The prediction could be further improved when lesion size (R2: 0.36, set 2 Table 2) and the degree of CST damaged (R2: 0.38, set 3, Table 2) were added.

Importantly, when using the set of features that included on top of the benchmark features the brain connectivity measures, the predictive model significantly outperformed all the others in terms of accuracy (R2: 0.68, set 4 Table 2). Finally, when removing CST asymmetry, which is rarely available, the prediction was still higher than benchmark features alone (R2: 0.46, set 5 Table 2). Advantageously, the present invention outperforms state-of-the-art methods both in term of specificity and sensibility.

The similarity in R2 between Set 3 and Set 5 demonstrates that CST asymmetry, which needs long and tedious DTI analyses, is equivalent to a full-brain analysis of the impact of the lesion on the network, which merely needs a binary image of the stroke lesion. Increased accuracy of prediction could instead be achieved only combining CST asymmetry and whole-brain network changes.

Table 3 illustrates that advantageously other prediction models than Ridge regression give similar values. Therefore, this shows that results are very robust over different prediction models.

Table 3: R2, specificity, sensitivity, and positive and negative predictive value for different prediction models on the set of features 4 with the best VIF threshold. PPV: positive predictive value; NNV: negative predictive value.

| Value | Ridge Regression | Multilinear Regression | Lasso | Elastic Net | Bayesian Ridge |
|---|---|---|---|---|---|
| $R^2$ | 0.68 | 0.68 | 0.67 | 0.66 | 0.51 |
| Specificity | 1.00 | 1.00 | 1.00 | 1.00 | 0.94 |
| Sensitivity | 0.94 | 0.94 | 0.94 | 0.94 | 0.82 |
| PPV | 1.00 | 1.00 | 1.00 | 1.00 | 0.93 |
| NPV | 0.94 | 0.94 | 0.94 | 0.94 | 0.84 |

While the embodiments have been described in conjunction with a number of embodiments, it is evident that many alternatives, modifications and variations would be or are apparent to those of ordinary skill in the applicable arts. Accordingly, this disclosure is intended to embrace all such alternatives, modifications, equivalents and variations that are within the scope of this disclosure.

## Claims

1. Computer-implemented method for generating a graph representing a virtually lesioned connectome of a patient after a stroke, the method comprising

   - providing structural data of the brain of the patient after the stroke;
   - segmenting said structural data to delineate lesioned voxels and non-lesioned voxels so as to generate a lesion map;
   - providing at least one connectome of a healthy reference brain, said connectome representing white matter fibers of the said healthy reference brain;
   - overlapping the lesion map on the connectome and removing each white matter fiber of the connectome passing through one lesioned voxel so as to generate a virtually lesioned connectome;
   - constructing a graph for each virtually lesioned connectome by parcellating the virtually lesioned connectome into N region of interest, each region of interest corresponding to a vertex of the graph, the edges connecting two vertices representing the fibers connecting two regions of interest;

2. Method according to claim 1, wherein a plurality of connectomes from healthy reference brains is provided, each connectome being used to generate one virtually lesioned connectome, and each virtually lesioned connectome is used to construct one graph.

3. Method according to any one of claims 1 or 2, wherein the method comprises

   - computing brain connectivity measures from the generated graph for instance binary graph measures and/or weighted graph measures, preferably binary graph measures chosen form the list comprising density, median degree, mean clustering coefficient, median clustering coefficient, mean flow coefficient, transitivity, modularity, mean eigenvector centrality, median eigenvector centrality, mean betweenness centrality, median betweenness centrality, preferably weighted graph measures preferably chosen form the list comprising algebraic connectivity, mean eigenvector centrality, median eigenvector centrality, modularity, global efficiency, participation coefficient, quasi-idempotence,

- preferably the brain connectivity measures comprise modularity weighted, participant coefficient, transitivity, median eigenvector centrality and mean eigenvector centrality weighted.

4. Method according to the preceding claim, wherein when several graphs are constructed, the method comprises:

- Averaging the shared connectivity measures among the graphs to obtain an average value for each shared connectivity measure;

5. Method according to any one of claims 1 to 4, wherein the structural data comprises data chosen from the list comprising MRI data such as T2-weighted, T1-weighted, diffusion-weighted, computerized tomography (CT).

6. Method according to any one of claims 1 to 5, wherein the segmenting step is performed automatically for instance by using an automated segmenting tool or manually by an operator.

7. Method according to any one of claims 1 to 6, wherein the lesioned map corresponds to three dimensions 3D map of zeros for non-lesioned voxels and ones for lesioned voxels.

8. Computer-implemented method for predicting the recovery of a patient after a determined period following a stroke, the method comprises

- providing a set of features measured from the patient after the stroke;
- providing a computational model configured for predicting the recovery of a patient after a determined period following a stroke;
- using said set of features as input for the computational model to calculate a recovery score as output to predict the recovery of the patient;

the method being **characterized in that** the set of features comprises brain connectivity measures, said brain connectivity measures being computed from at least one graph generated by a method according to any one of claims 1 to 7.

9. Method according to the preceding claim, wherein the method further comprises

- Selecting a group of features among the set of connectivity measures, the selection being carried out using a selection tool such as variance inflating factors (VIF) LASSO, or hierarchical clustering, preferably variance inflating factors.

10. Method according to any one of claims 8 to 9, wherein the recovery score is chosen among the list comprising Fugl-Meyer Assessment (FMA) scale, Stroke Impact Assessment Scale, the Chedoke-McMaster Stroke Assessment, or the Motor Assessment Scale, preferably Fugl-Meyer Assessment scale.

11. Method according to any one of claims 8 to 10, wherein computational model is chosen among the list comprising ridge regression, Multilinear regression, Lasso, Elastic Net, Bayesian Ridge, preferably ridge regression.

12. Method according to any one of claims 8 to 11, wherein the input for the computational model correspond to a set of standardized features to obtain a mean of 0 and a standard deviation of 1 for each feature.

13. Method according to any one of claims 8 to 12, wherein the set of features further comprises features chosen from the list comprising patient's age, initial motor impairment, lesion volume, cortico-spinal tract asymmetry.

14. Device comprising means for carrying out the method of any one of claims 1 to 13, preferably the device is a computer device.

15. Computer program comprising instructions which, when the program is executed by a computer, cause the computer to carry out the method of any one of claims 1 to 13.

Figure 1

Figure 2

EP 4 336 517 A1

Europäisches Patentamt
European Patent Office
Office européen des brevets

# EUROPEAN SEARCH REPORT

Application Number

EP 22 19 4751

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KUCEYESKI AMY ET AL: "Structural connectome disruption at baseline predicts 6-months post-stroke outcome : The Connectome and Recovery From Stroke", HUMAN BRAIN MAPPING, vol. 37, no. 7, 26 March 2016 (2016-03-26), pages 2587-2601, XP093027196, ISSN: 1065-9471, DOI: 10.1002/hbm.23198 * Chapter METHODS – Subjects and Data – Image Procerssing and the NeMo Tool – Predicting Six-Month APMAC Scores: Global, Regional and Pairwise Level Models * ----- | 1-15 | INV. G16H50/30 G16H50/50 |
| X | HALAI AJAY D ET AL: "Investigating the effect of changing parameters when building prediction models for post-stroke aphasia", NATURE HUMAN BEHAVIOUR, NATURE PUBLISHING GROUP UK, LONDON, vol. 4, no. 7, 20 April 2020 (2020-04-20), pages 725-735, XP037194913, DOI: 10.1038/S41562-020-0854-5 [retrieved on 2020-04-20] * point "Neuroimaging preprocessing"; page 732 * * point "DWI"; page 732 * * page 725 – page 729 * ----- -/-- | 1-15 | |

TECHNICAL FIELDS SEARCHED (IPC)

G16H

The present search report has been drawn up for all claims

| Place of search | Date of completion of the search | Examiner |
|---|---|---|
| Munich | 28 February 2023 | Eichenauer, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

13

**EP 4 336 517 A1**

Europäisches Patentamt
European Patent Office
Office européen des brevets

# EUROPEAN SEARCH REPORT

Application Number

EP 22 19 4751

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HALAI AJAY D. ET AL: "Predicting the pattern and severity of chronic post-stroke language deficits from functionally-partitioned structural lesions", NEUROIMAGE: CLINICAL, vol. 19, 1 January 2018 (2018-01-01), pages 1-13, XP093025389, ISSN: 2213-1582, DOI: 10.1016/j.nicl.2018.03.011 Retrieved from the Internet: URL:https://www.sciencedirect.com/science/article/pii/S2213158218300810/pdfft?md5=5785b48458cbd279e5c13156d07d018b&pid=1-s2.0-S2213158218300810-main.pdf> * chapter "2. Materials and methods" * ----- | 1-15 | |
| X | MESKALDJI DJALEL-EDDINE ET AL: "Prediction of long-term memory scores in MCI based on resting-state fMRI", NEUROIMAGE: CLINICAL, vol. 12, no. 2, 11 October 2016 (2016-10-11), pages 785-795, XP093024473, DOI: https://doi.org/10.1016/j.nicl.2016.10.004 Retrieved from the Internet: URL:https://www.sciencedirect.com/science/article/pii/S2213158216301851> * section "2. Methods" * ----- -/-- | 1-15 | **TECHNICAL FIELDS SEARCHED (IPC)** |

The present search report has been drawn up for all claims

| Place of search | Date of completion of the search | Examiner |
|---|---|---|
| Munich | 28 February 2023 | Eichenauer, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

14

Europäisches
Patentamt

European
Patent Office

Office européen
des brevets

# EUROPEAN SEARCH REPORT

Application Number

EP 22 19 4751

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PIRONDINI ELVIRA ET AL: "Post-stroke reorganization of transient brain activity characterizes deficits and recovery of cognitive functions", NEUROIMAGE, vol. 255, 1 July 2022 (2022-07-01), page 119201, XP093025418, AMSTERDAM, NL ISSN: 1053-8119, DOI: 10.1016/j.neuroimage.2022.119201 * abstract * * chapter "2. Material and methods" * ----- | 1-15 | |
| X | LEE J ET AL: "Differential early predictive factors for upper and lower extremity motor recovery after ischaemic stroke", EUROPEAN JOURNAL OF NEUROLOGY, RAPID SCIENCE PUBLISHERS, GB, vol. 28, no. 1, 27 September 2020 (2020-09-27), pages 132-140, XP072032307, ISSN: 1351-5101, DOI: 10.1111/ENE.14494 * chapter "Methods" * ----- | 1-15 | |
| A | Anonymous: "Connectome", Wikipedia, 2 June 2022 (2022-06-02), pages 1-18, XP093023859, Retrieved from the Internet: URL:https://en.wikipedia.org/w/index.php?title=Connectome&oldid=1091121125 [retrieved on 2023-02-14] * the whole document * ----- -/-- | 1-15 | TECHNICAL FIELDS SEARCHED (IPC) |

The present search report has been drawn up for all claims

| Place of search | Date of completion of the search | Examiner |
|---|---|---|
| Munich | 28 February 2023 | Eichenauer, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 336 517 A1**

Europäisches Patentamt
European Patent Office
Office européen des brevets

# EUROPEAN SEARCH REPORT

Application Number

**EP 22 19 4751**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Anonymous: "Variance inflation factor", Wikipedia, 28 January 2022 (2022-01-28), pages 1-5, XP093023866, Retrieved from the Internet: URL:https://en.wikipedia.org/w/index.php?title=Variance_inflation_factor&oldid=1068481283 [retrieved on 2023-02-14] * the whole document * ----- | 9 | |
| A | Anonymous: "White matter", Wikipedia, 2 May 2022 (2022-05-02), pages 1-6, XP093024747, Retrieved from the Internet: URL:https://en.wikipedia.org/w/index.php?title=White_matter&oldid=1085710917 [retrieved on 2023-02-16] * the whole document * ----- | 1-15 | |

TECHNICAL FIELDS SEARCHED (IPC)

The present search report has been drawn up for all claims

| Place of search | Date of completion of the search | Examiner |
|---|---|---|
| Munich | 28 February 2023 | Eichenauer, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

16